# EUROPÄISCHE PATENTSCHRIFT

(11) **EP 2 704 680 B1**
(45) Veröffentlichungstag und Bekanntmachung des Hinweises auf die Patenterteilung: **25.03.2015**
(21) Anmeldenummer: 13721601.6
(22) Anmeldetag: 02.04.2013
(51) Int. Cl.: A61G 10/02, B01D 53/30, A63B 24/00, A61B 5/08

(54) **VERFAHREN ZUR STEUERUNG EINES TRAININGSGERÄTS**
METHOD FOR CONTROLLING A TRAINING DEVICE
PROCÉDÉ DE COMMANDE D'UN APPAREIL D'ENTRAÎNEMENT

(30) Priorität: 30.03.2012 EP 12162522; 27.07.2012 EP 12178249
(43) Veröffentlichungstag der Anmeldung: 12.03.2014
(73) Patentinhaber: NL NANOMED Limited, 3101 Limassol (CY)
(72) Erfinder: BENDZKO, Peter, 12623 Berlin (DE); SCHULZ, Jörg, 13127 Berlin (DE); ALBAKOV, Adam, Moskau 115172 (RU)
(74) Vertreter: Sokolowski, Fabian
(86) Internationale Anmeldenummer: PCT/EP2013/056900
(87) Internationale Veröffentlichungsnummer: WO 2013/144366

(56) Entgegenhaltungen:
- EP-A1- 1 825 888
- EP-A2- 1 055 393
- WO-A1-02/16010
- WO-A2-2011/087488
- DE-A1- 4 406 286
- DE-A1- 10 257 155
- DE-A1-102007 039 124
- DE-A1-102007 052 776
- JP-A- H10 216 455
- JP-A- 2003 105 995
- US-A1- 2004 186 389

## Beschreibung

Die vorliegende Erfindung betrifft ein Verfahren zur Steuerung eines Trainingsgerätes gemäß dem Oberbegriff des Anspruchs 1.

Aus der DE 10 2004 034640 A1 ist ein Verfahren zur systemischen Biokorrektur eines Organismus bekannt. Dabei wird auch beschrieben, ein Laufband oder ein Ergometer durch den jeweiligen respiratorischen Quotienten eines Benutzers zu steuern. Ziel dieser Steuerung ist die Einstellung eines respiratorischen Quotienten von 0,70 bis 0,76 bei Pulsfrequenzen zwischen 120 und 150 Schlägen pro Minute. Derart hohe Pulsfrequenzen werden nur dann erreicht, wenn ein Leistungsäquivalent des eingesetzten Laufbands oder Fahrrads oberhalb von 90 Watt liegt. Der Begriff "Leistungsäquivalent" bezeichnet dabei die Leistung, die ein Benutzer auf einem Laufband bzw. einem Ergometer erbringen muss, um eine bestimmte Geschwindigkeit zu erreichen.

Die WO 02/20136 A1 beschreibt ein Verfahren und ein System zur Simulierung der Atmosphäre in unterschiedlichen Höhen über dem Meeresboden. Dabei wird entweder ein Sauerstoffkonzentrator oder ein Stickstoffgenerator eingesetzt, um die Sauerstoffkonzentration in einem Raum auf die einer gewünschten Höhe über dem Meeresboden entsprechende Sauerstoffkonzentration einzustellen. Im Rahmen der Lehre dieser internationalen Patentanmeldung ist es stets vorgesehen, mittels eines Ventilators zusätzliche Frischluft in den Raum einzuführen.

Aus der DE 102 57 155 A1 ist ein Verfahren zum Einstellen einer Raumluft in einem Raum bekannt. Bei diesem Verfahren soll der Sauerstoffanteil der Raumluft auf weniger als 20,9 Volumenprozent (also auf weniger als den gewöhnlichen Sauerstoffanteil) abgesenkt werden. Dies wird durch das Mischen von Stickstoff und Frischluft erreicht, wobei das Gemisch aus Stickstoff und Frischluft dem Raum, dessen Atmosphäre anzupassen ist, zugeführt wird.

Aus der WO 97/03631 A1 ist ein Raumsystem zur Einstellung einer hypoxischen Atmosphäre (also einer Atmosphäre mit einem Sauerstoffgehalt, der unter dem normalen Sauerstoffgehalt von Luft liegt) bekannt. Zur Herstellung der hypoxischen Raumatmosphäre wird ein sogenannter Hypoxikator eingesetzt, der den Anteil von Sauerstoff an der Raumluft reduziert.

EP 1 825 888 A1, DE 2007 039 124 A1 und DE 10 2007 052 776 A1 offenbaren Verfahren zur Regelung von Trainingsgeräten.

DE 44 06 286 A1, EP 1 055 393 A2 und US 2004/186389 A1 offenbaren Verfahren zur Bestimmung von respiratorischen Kennwerte.

Der vorliegenden Erfindung liegt die Aufgabe zugrunde, ein gegenüber den aus dem Stand der Technik bekannten Verfahren zur Steuerung eines Trainingsgeräts verbessertes Verfahren zur Steuerung eines Trainingsgeräts bereitzustellen.

Diese Aufgabe wird durch ein Verfahren mit den Merkmalen des Anspruchs 1 gelöst.

Ein derartiges Verfahren zur Steuerung eines Trainingsgeräts umfasst die Schritte des Bereitstellens einer definierten Raumatmosphäre in einem Trainingsraum, in welchem sich das Trainingsgerät befindet, der Bestimmung eines respiratorischen Quotienten eines das Trainingsgerät in dem Trainingsraum benutzenden Probanden und des Verwendens des respiratorischen Quotienten des Probanden als Eingangsgröße zur Steuerung des Trainingsgeräts. Dabei bezieht sich das beanspruchte Verfahren nicht auf Verfahren zur therapeutischen Behandlung des menschlichen oder tierischen Körpers.

Das erfindungsgemäß beanspruchte Verfahren zeichnet sich dadurch aus, das die Raumatmosphäre in dem Trainingsraum einen konstanten Sauerstoffanteil aufweist, der im Bereich von 22 bis 32 Volumenprozent, insbesondere von 23 bis 31 Volumenprozent, insbesondere von 24 bis 30 Volumenprozent, insbesondere von 25 bis 29 Volumenprozent und ganz besonders von 26 bis 28 Volumenprozent liegt. Das Verfahren zeichnet sich ferner dadurch aus, dass ein vorgebbares Leistungsäquivalent des Trainingsgeräts in einem Bereich von 50 bis 85 Watt in Abhängigkeit des respiratorischen Quotienten des Probanden derart variiert wird, dass sich der respiratorische Quotient des Probanden auf einen Wert in einem Bereich von 0,77 bis 0,85 einstellt.

Das Leistungsäquivalent des Trainingsgerätes lässt sich über verschiedene Leistungsparameter des Trainingsgerätes einstellen und gibt diejenige Leistung an, die ein Proband erbringen muss, um auf dem Trainingsgerät bei den gewählten Einstellungen des Trainingsgeräts eine bestimmte Geschwindigkeit zu erreichen. Bei dem Trainingsgerät kann es sich beispielsweise um ein Laufband handeln. Durch Einstellung des Neigungswinkels und der Geschwindigkeit des Laufbandes kann die Leistung, die ein Proband, der auf dem Laufband läuft, erbringen muss, eingestellt werden. Das Trainingsgerät kann auch ein Ergometer sein. Dann wird das Leistungsäquivalent durch die Leistung definiert, die ein Proband benötigt, um eine definierte Geschwindigkeit unter den gewählten Einstellungen zu erreichen. Grundsätzlich sind beliebige Trainingsgeräte einsetzbar, sofern sich ihr Leistungsäquivalent beispielsweise durch Einstellung einer Federkraft oder einer magnetischen Kraft einstellen lässt.

Bei dem oben beschriebenen, aus dem Stand der Technik bekannten Verfahren zur Steuerung eines Trainingsgerätes ist eine Änderung des Sauerstoffanteils in der eingeatmeten Luft des das Trainingsgerät benutzenden Probanden vorgesehen. Beim vorliegenden Verfahren ist indes ein konstanter Sauerstoffanteil vorgesehen, der im hyperoxischen Bereich liegt beträgt. Als "konstant" wird der Sauerstoffanteil angesehen, wenn er innerhalb des vorgenannten Bereiches um bis zu 1 Volumenprozent, insbesondere um bis zu 0,5 Volumenprozent, insbesondere um bis zu 0,2 Volumenprozent und ganz besonders um bis zu 0,1 Volumenprozent schwankt. Diese Schwankungen können sich durch den Sauerstoffverbrauch des Probanden und einer CO₂-Anreicherung in der Raumatmosphäre durch die Ausatemluft des Probanden ergeben.

Mit dem vorgebbaren Leistungsäquivalent des Trainingsgerätes im Bereich von 50 bis 85 Watt, insbesondere im Bereich von 55 bis 80 Watt, insbesondere von 60 bis 75 Watt und ganz besonders im Bereich von 65 bis 70 Watt kann erreicht werden, dass eine Herzfrequenz bzw. eine Pulsfrequenz des Probanden bei etwa 70 bis 90 Schlägen pro Minute, insbesondere bei 75 bis 85 Schlägen pro Minute und ganz besonders bei 78 bis 83 Schlägen pro Minute liegt.

Bei aus dem Stand der Technik bekannten Verfahren wird stets eine deutlich höhere Pulsfrequenz des Probanden erreicht, die sich nachteilig auf den Probanden und dessen Metabolismus auswirkt. Diese höhere Herzfrequenz bzw. Pulsfrequenz wird durch höhere Leistungsäquivalente des eingesetzten Trainingsgerätes hervorgerufen, die regelmäßig im Bereich von 90 bis 120 Watt liegen.

Bei einem respiratorischen Quotienten von 0,77 bis 0,85 ergibt sich eine vorteilhafte Umstellung des Metabolismus des Probanden hin zu einem verstärkten Fettabbau. Das vorliegend beanspruchte Verfahren zur Steuerung eines Trainingsgerätes eignet sich also indirekt zur Maximierung des Fettabbaus des Probanden, der das Trainingsgerät benutzt. Weitere Parameter, die den Fettabbau des Probanden maximieren, sind die hyperoxische Raumatmosphäre und die durch das vorgegebene Leistungsäquivalent des Trainingsgerätes bedingte niedrige Herzfrequenz bzw. Pulsfrequenz des Probanden. Bei aus dem Stand der Technik bekannten Verfahren war man davon ausgegangen, dass eine Pulsfrequenz von mehr als 120 Schlägen pro Minute erforderlich ist, um eine Metabolismusumstellung des Probanden zu erreichen. Überraschenderweise hat sich jedoch gezeigt, dass niedrige Pulsfrequenzen im oben angegebenen Bereich deutlich geeigneter sind, um für eine Umstellung des Metabolismus des Probanden zu sorgen. Gleichzeitig konnte überraschenderweise gezeigt werden, dass auch eine hyperoxische Raumatmosphäre weitaus besser geeignet ist, um eine Metabolismusumstellung im Probanden zu erreichen als im Stand der Technik häufig verwendete hypooxische Raumatmosphären.

Aus dem Stand der Technik gibt es damit keine Anhaltspunkte, die Steuerung des Trainingsgeräts in den oben erläuterten Parameterbereichen vorzunehmen.

Der Sauerstoffanteil in der Raumatmosphäre muss zudem überraschenderweise nicht in Abhängigkeit des respiratorischen Quotienten des Probanden angepasst werden, um eine geeignete Metabolismusumstellung des Probanden zu erreichen. Vielmehr ist ein konstanter Sauerstoffanteil im hyperoxischen Bereich eine weitaus besser geeignete Ausgangsgröße, um eine Metabolismusumstellung beim Probanden zu erreichen.

Durch die technischen Parameter des Sauerstoffanteils in der Raumatmosphäre, des Leistungsäquivalents des Trainingsgeräts und der Anpassung des Leistungsäquivalents in Abhängigkeit des respiratorischen Quotienten des Probanden lässt sich durch das beanspruchte Verfahren zur Steuerung des Trainingsgerätes als Nebeneffekt eine Umstellung des Metabolismus des Probanden hin zu einer verstärkten Fettmetabolisierung erreichen. Gleichzeitig wird die ansonsten vorrangig stattfindende Zuckermetabolisierung reduziert.

In einer alternativen Ausgestaltung des Verfahrens wird das Leistungsäquivalent des Trainingsgerätes erhöht, wenn der respiratorische Quotient des Probanden größer als 0,85 ist. Eine derartige Erhöhung des Leistungsäquivalents kann einmal oder auch mehrmals pro Trainingseinheit durchgeführt werden.

In einer Variante weist eine Trainingseinheit eine Zeitdauer von etwa 30 bis 90 Minuten, insbesondere von 45 bis 75 Minuten und ganz besonders von 55 bis 65 Minuten (also rund 60 Minuten) auf.

In einer weiteren alternativen Ausgestaltung des Verfahrens wird das Leistungsäquivalent des Trainingsgerätes erniedrigt, wenn der respiratorische Quotient kleiner als 0,77 ist. Durch eine derartige Einstellung wird erreicht, dass der respiratorische Quotient des Probanden auf einen Wert im Bereich von 0,77 bis 0,85 gehalten wird.

In einer weiteren alternativen Verfahrensausgestaltung wird das Leistungsäquivalent des Trainingsgerätes zumindest während eines Zeitraums von 5 Minuten, insbesondere von 10 Minuten, insbesondere von 15 Minuten und ganz besonders von 30 Minuten oder mehr konstant gehalten. Durch ein derartiges Konstanthalten des Leistungsäquivalents können kurzfristige Schwankungen im respiratorischen Quotienten des Probanden ausgeglichen werden, die anderenfalls zu einer unerwünschten Fehlsteuerung des Trainingsgerätes führen könnten. Typischerweise erfolgt jedoch etwa alle fünf Minuten eine Neuanpassung des Leistungsäquivalents des Trainingsgerätes in Abhängigkeit des respiratorischen Quotienten des Probanden, um das Leistungsäquivalent möglichst zeitnah an den respiratorischen Quotienten des Probanden anzupassen.

In einer weiteren alternativen Verfahrensausgestaltung wird zur Bestimmung des respiratorischen Quotienten des Probanden eine Messung der CO₂-Konzentration in der Ausatemluft und optional in der Einatemluft mittels eines maskenlosen Messsystems durchgeführt. Gleichzeitig mit der Messung der CO₂-Konzentration in der Ausatemluft des Probanden kann auch die Sauerstoffkonzentration in der Ausatemluft und optional in der Einatemluft des Probanden bestimmt werden. Dies geschieht typischerweise über dem Fachmann allgemein bekannte CO₂-Sensoren und Sauerstoffsensoren. Aus dem konstanten Sauerstoffanteil in der Raumatmosphäre und dem Sauerstoffanteil in der Ausatemluft des Probanden lässt sich dann die von dem Probanden aufgenommene Sauerstoffmenge bestimmen. Hierzu wird typischerweise ein Durchflusssensor eingesetzt, um die Menge der von dem Probanden ausgeatmeten Ausatemluft pro Zeiteinheit zu erfassen.

In einer Variante des Verfahrens erfolgt die Messung der CO₂-Konzentration und/oder der Sauerstoffkonzentration in der Ausatemluft des Probanden in Intervallen. Das heißt, es wird keine kontinuierliche Messung der CO₂-Konzentration bzw. der Sauerstoffkonzentration durchgeführt, sondern eine diskontinuierliche Messung. Dies ermöglicht es dem Probanden, das Trainingsgerät in bestimmten Zeitabschnitten ohne jegliche Verbindung zum Messsystem zu benutzen. Lediglich dann, wenn eine Messung des CO₂-Anteils in der Ausatemluft (also der CO₂-Konzentration in der Ausatemluft) erfolgen soll, muss der Proband eine geeignete Verbindung mit dem Messsystem herstellen.

In einer Variante des Verfahrens atmet der Proband die Ausatemluft zumindest einer Ausatmung in ein Mundstück aus, das mit einem CO₂-Sensor verbunden ist, um die CO₂-Konzentration in der Ausatemluft zu messen. Die Verbindung zwischen dem Mundstück und dem CO₂-Sensor wird typischerweise durch einen Schlauch hergestellt. Optional kann das Mundstück auch mit einem Sensor verbunden sein, der sowohl die CO₂-Konzentration als auch die Sauerstoffkonzentration in der Ausatemluft des Probanden bestimmen kann. Typischerweise wird nicht nur die Ausatemluft einer einzigen Ausatmung einer CO₂-Konzentrationsmessung unterzogen, sondern die Ausatemluft von etwa 8 bis 15, insbesondere 10 bis 12 Atemzyklen.

In einer weiteren alternativen Verfahrensausgestaltung wird eine Herzfrequenz des Probanden bestimmt und als zusätzliche Größe zur Steuerung des Trainingsgerätes herangezogen. Auf diese Weise lässt sich beispielsweise verhindern, dass das Leistungsäquivalent des Trainingsgerätes weiter erhöht wird, auch wenn aufgrund des ermittelten respiratorischen Quotienten des Probanden eine Erhöhung des Leistungsäquivalentes durchaus gerechtfertigt wäre. In dieser Variante wird das Leistungsäquivalent des Trainingsgerätes also nicht nur in Abhängigkeit des respiratorischen Quotienten des Probanden, sondern auch in Abhängigkeit der Herzfrequenz des Probanden variiert.

Es wird auch ein Computerprogrammprodukt mit einem Computerprogramm offenbart, das einen Programmcode zur Bestimmung des respiratorischen Quotienten und zur Steuerung des Trainingsgerätes gemäß dem zuvor beschriebenen Verfahren und dessen Verfahrensalternativen aufweist. Dabei sorgt der Programmcode des Computerprogramms zur Durchführung dieser Verfahrensschritte, wenn das Computerprogramm auf einem Computer ausgeführt wird.

Es wird ferner eine Vorrichtung zur Bereitstellung einer definierten Raumatmosphäre in einem Trainingsraum mit den nachfolgend näher erläuterten Merkmalen offenbart. Diese Vorrichtung dient zur Bereitstellung einer hyperoxischen Raumatmosphäre mit einem Sauerstoffanteil von 22 bis 32 Volumenprozent. Die Vorrichtung umfasst eine Luftzerlegungsanlage, die als Gaszuführeinrichtung für einen Trainingsraum dient. Die Luftzerlegungsanlage kann beispielsweise durch Öffnen entsprechender Ventile mit dem Trainingsraum in Strömungsverbindung gebracht werden. Durch das Schließen derartiger Ventile ist es in einer Variante zudem möglich, die Strömungsverbindung zwischen der Luftzerlegungsanlage und dem Trainingsraum zu unterbrechen. Im bestimmungsgemäßen Betrieb zerlegt die Luftzerlegungsanlage die ihr zugeführte Luft in einen sauerstoffangereicherten Volumenstrom und in einen stickstoffangereicherten Volumenstrom. Die der Luftzerlegungsanlage zugeführte Luft kann beispielsweise Druckluft sein.

Die Vorrichtung zeichnet sich dadurch aus, dass die Luftzerlegungsanlage die einzige externe Gaszuführeinrichtung des Trainingsraumes ist. Insbesondere weist der Trainingsraum keine Ventilatoren zur Zuführung von Frischluft auf. Ferner ist auch keine Frischluftzufuhr in den sauerstoffangereicherten Volumenstrom und den stickstoffangereicherten Volumenstrom der Luftzerlegungsanlage vorgesehen. Vielmehr dienen die beiden Volumenströme der Luftzerlegungsanlage als einzige und exklusive Gaszufuhren für den Trainingsraum.

Gegenüber aus dem Stand der Technik bekannten Systemen zur Bereitstellung einer definierten Raumatmosphäre hat die vorliegend beschriebene Vorrichtung den großen Vorteil, dass sie in energieoptimierter Weise arbeiten kann. Denn es wird keine Zusatzenergie für eine in bekannten Raumluftsystemen erforderliche Frischluftaufbereitung benötigt. Vielmehr muss lediglich die für die Luftzerlegungsanlage benötigte Energie aufgewendet werden. Durch eine entsprechende Mischung des sauerstoffangereicherten Volumenstroms und des stickstoffangereicherten Volumenstroms der Luftzerlegungsanlage kann ein Atemgas bereitgestellt werden, das einen Sauerstoffanteil von 22 bis 32 Volumenprozent aufweist. Eine zusätzliche Zufuhr von Stickstoff oder Sauerstoff aus externen Quellen ist nicht erforderlich. Damit ist es auch nicht erforderlich, zusätzliche Trocknungs-, Filtrations- oder Temperierungsvorrichtungen für externe Gasquellen vorzusehen, wie dies bei aus dem Stand der Technik bekannten Raumluftsystemen regelmäßig der Fall ist.

In einer alternativen Ausgestaltung ist die Vorrichtung dazu vorgesehen und eingerichtet, dass in ihrem bestimmungsgemäßen Betrieb sowohl ein Anteil des sauerstoffangereicherten Volumenstroms als auch ein Anteil des stickstoffangereicherten Volumenstroms dem Trainingsraum zugeführt wird. Dies kann über separate Leitungen erfolgen. Alternativ ist es auch möglich, den sauerstoffangereicherten Volumenstrom und den stickstoffangereicherten Volumenstrom einer vor dem Trainingsraum angeordneten Mischkammer zuzuführen, in der beide Volumenströme in dem gewünschten Verhältnis miteinander vermischt werden. In den Trainingsraum wird dann bereits vorgemischte Luft mit dem gewünschten Sauerstoffanteil im hyperoxischen Bereich zugeführt.

In einer alternativen Ausgestaltung weist der sauerstoffangereicherte Volumenstrom einen Sauerstoffanteil von 22 bis 45 Volumenprozent, insbesondere von 23 bis 44 Volumenprozent, insbesondere von 24 bis 43 Volumenprozent, insbesondere von 25 bis 40 Volumenprozent und ganz besonders von 30 bis 35 Volumenprozent auf. Im Grenzfall, bei dem ein sauerstoffangereicherter Volumenstrom mit einem Sauerstoffanteil von 22 Volumenprozent verwendet wird und eine Raumatmosphäre in dem Trainingsraum mit einem Sauerstoffanteil von 22 Volumenprozent eingestellt werden soll, muss der Anteil des stickstoffangereicherten Volumenstroms an dem dem Trainingsraum zugeführten Gasgemisch auf Null herabgesetzt werden. Das heißt, die gesamte Raumatmosphäre in dem Trainingsraum muss durch den zugeführten sauerstoffangereicherten Volumenstrom ersetzt werden. Dies ist ein verhältnismäßig langwieriger Prozess, so dass zumindest beim Beginn des Einstellens einer definierten Raumatmosphäre höhere Sauerstoffanteile im sauerstoffangereicherten Volumenstrom bevorzugt werden.

In einer weiteren Variante weist der stickstoffangereicherte Volumenstrom einen Stickstoffanteil von 90 bis 100 Volumenprozent, insbesondere von 92 bis 99 Volumenprozent, insbesondere von 93 bis 98 Volumenprozent, insbesondere von 94 bis 97 Volumenprozent und ganz besonders von 95 bis 96 Volumenprozent auf. Sofern der Stickstoffanteil unter 100 Volumenprozent liegt, finden sich in dem stickstoffangereicherten Volumenstrom - wie auch im sauerstoffangereicherten Volumenstrom - die sonstigen Gasbestandteile, die in der der Luftzerlegungsanlage zugeführten Luft enthalten sind. Hierbei handelt es sich beispielsweise um CO₂, Edelgase etc.

In einer weiteren alternativen Ausgestaltung weist die Luftzerlegungsanlage Vorrichtungen zur Filterung und Trocknung der ihr zugeführten Luft auf. Bei der der Luftzerlegungsanlage zugeführten Luft kann es sich beispielsweise um Druckluft handeln. Alternativ ist es auch möglich, dass der Luftzerlegungsanlage Raumluft aus dem Trainingsraum zugeführt wird (Umluftbetrieb). Ferner ist es möglich, dass eine Mischung aus Druckluftzufuhr und Raumluftzufuhr (kombinierter Umluftbetrieb) vorgesehen ist.

Da nur eine einzige Gaszuführung zum Trainingsraum vorgesehen ist, ist es auch nur erforderlich, eine einzige Vorrichtung zur Filterung und Trocknung von Luft vorzusehen. Das reduziert den apparativen Aufwand der vorliegend beanspruchten Vorrichtung zur Bereitstellung einer definierten Raumatmosphäre gegenüber den aus dem Stand der Technik bekannten Raumluftsystemen.

In einer Ausführungsvariante weist die Vorrichtung einen CO₂-Abscheider zur Reduzierung des CO₂-Anteils in der Raumatmosphäre auf. Ein derartiger CO₂-Abscheider kann beispielsweise im Trainingsraum angeordnet sein oder der Luftzerlegungsanlage in Strömungsrichtung vorgeschaltet sein. Letztere Variante bietet sich insbesondere dann an, wenn das Raumluftsystem im Umluftbetrieb betrieben werden soll.

In einer weiteren Variante kann die Vorrichtung eine Umluftanlage umfassen, die insbesondere im Trainingsraum selbst angeordnet ist. Eine derartige Umluftanlage, die beispielsweise als Ventilator ausgestaltet sein kann, dient zur besseren Vermischung der in dem Trainingsraum vorhandenen Gase. Dadurch lässt sich schneller eine definierte Raumatmosphäre in dem Trainingsraum einstellen.

In einer alternativen Ausgestaltung werden eine Trainingsmethodik und ein individuelles Trainingsprogramm angesetzt.

Kardioprogramme, welche Leistung über die Herzfrequenz steuern, gehören zum Stand der Technik. Wenn eine Herzfrequenzsteuerung nicht möglich ist (Gerät besitzt keine Kardiosteuerung oder die Herzfrequenzbestimmung ist nicht fehlerfrei möglich), kann mit den RQ-Werten die Leistung des Trainingsgerätes auch direkt gesteuert werden.

Die Herzfrequenzsteuerung ist auch deshalb wichtig, weil die RQ-Messung erst nach etwa 10 Minuten, wenn im Organismus ein erstes Gleichgewicht (steady state) vorliegt, zur Korrektur der Leistung herangezogen werden kann.

Die Steuerung erfolgt automatisch. Das zeitliche Intervall der Messwerterfassung und die Korrekturgrößen richten sich nach der Abweichung der RQ-Werte vom Sollbereich und sind deshalb variabel.

Die Logik der Beeinflussung des Herzfrequenzgrenzbereiches durch die gemessenen RQ-Werte ist eine Neuerung.

Vorteilhafterweise werden vor, während und nach der Belastung außer dem RQ-Wert die Herzfrequenz, die momentane Leistung, die Sauerstoffsättigung und/oder der Blutdruck (optional) der Person (des Probanden) gemessen. Die Herzfrequenz wird vorzugsweise telemetrisch laufend gemessen. Es ist im Stand der Technik nicht beschrieben, dass Herzfrequenzbereiche durch gemessene RQ- Werte beeinflusst werden können. Die Korrekturen bzw. die Steuerung erfolgt vorzugsweise automatisch und kontinuierlich.

Für die Messung des respiratorischen Quotienten wird vorzugsweise ein maskenloses Messsystem verwendet. Es ist zudem bevorzugt, dass die Einstellungen, die am Trainingsgerät vorzunehmen sind, automatisch oder manuell erfolgen.

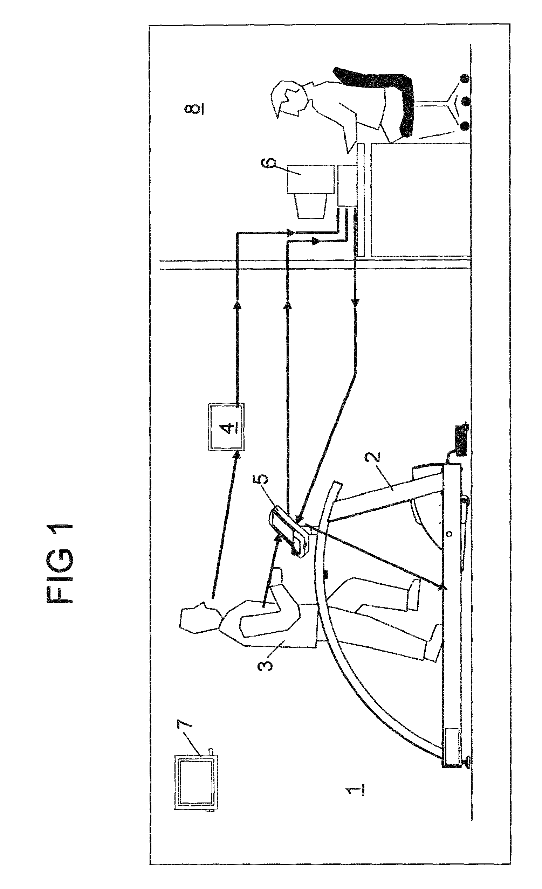

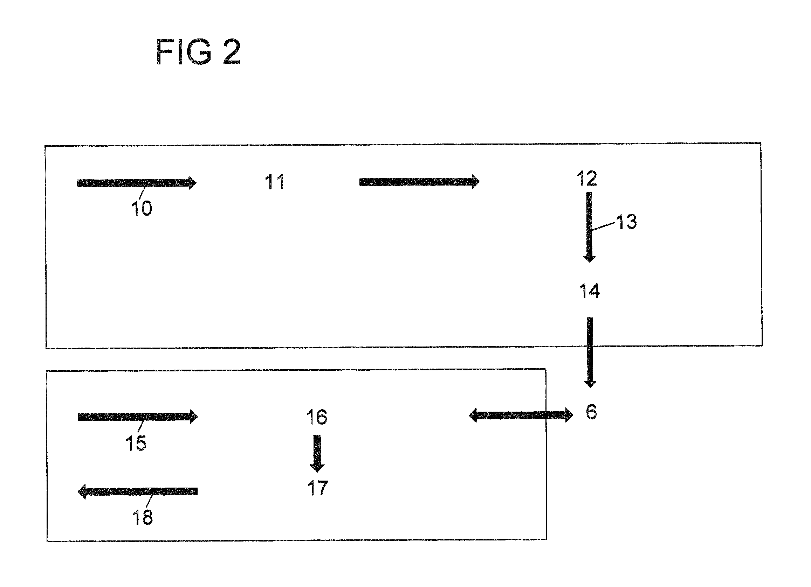

Ein spezielles Messmodul erfasst in einem Datenprotokoll synchron und atemzuggenau die Sauerstoff- und Kohlendioxidkonzentration der Inspirations- und Exspiritationsluft. Zusammen mit den erfassten Herzfrequenz- (Hf-) und Ergometerleistungsdaten werden diese nach einem neu entwickelten Algorithmus für die RQ gestützte Steuerung verarbeitet (siehe hierzu auch Figuren 1 und 2).

Bei dem Trainingsgerät kann es sich insbesondere um ein Ergometer oder ein sonstiges Trainingsgerät zum Training im bevorzugt aeroben Bereich handeln (z. B. Laufband, Fahrradergometer oder Crosstrainer). Es ist insbesondere bevorzugt, dass es sich hierbei um ein steuerbares Ergometer oder Trainingsgerät handelt.

Weitere Details und Ausführungsvarianten der beschriebenen Verfahren und der beschriebenen Vorrichtungen werden anhand beigefügter Figuren näher erläutert. Es zeigen:
- Figur 1: eine schematische Darstellung eines Trainingsraum, in dem ein gesteuertes Trainingsgerät angeordnet ist,
- Figur 2: ein Ablaufdiagramm für ein Verfahren zur Steuerung eines Trainingsgeräts,
- Figur 3: ein erstes Ausführungsbeispiel eines Raumluftsystems,
- Figur 4: ein zweites Ausführungsbeispiel eines Raumluftsystems,
- Figur 5: ein drittes Ausführungsbeispiel eines Raumluftsystems und
- Figur 6: ein viertes Ausführungsbeispiel eines Raumluftsystems.

Die Figur 1 zeigt eine beispielhafte Darstellung eines Trainingsraumes 1, in dem ein Laufband 2 als Trainingsgerät angeordnet ist. Das Laufband 2 gibt eine bestimmte Geschwindigkeit vor, die ein Proband 3, der das Laufband benutzt, einhalten muss, um sich trotz der Bewegung des Laufbands 2 in etwa an derselben Stelle des Laufbands 2 zu befinden. Dazu muss der Proband 2 eine bestimmte Leistung aufbringen. Die Geschwindigkeit, die das Laufband 2 vorgibt, stellt dabei - in Zusammenschau mit einem Neigungswinkel, den das Laufband 2 einnehmen kann - ein Leistungsäquivalent dar, das derjenigen Leistung entspricht, die der Proband 3 aufbringen muss.

In regelmäßigen Zeitabständen atmet der Proband 3 Ausatemluft in ein Messmodul 4 aus, das einen Sauerstoffsensor und einen Kohlendioxidsensor aufweist und zur Bestimmung der Sauerstoffkonzentration und der C02-Konzentration in der Ausatemluft des Probanden dient. Die Sauerstoffkonzentration und C02-Konzentration kann dabei atemzuggenau vom Messmodul 4 bestimmt werden.

Der Proband 3 trägt zudem einen nicht dargestellten Herzfrequenzmesser, der die Herzfrequenz des Probanden 3 drahtlos an einen Monitor 5 des Laufbands 2 überträgt.

Die vom Messmodul 4 ermittelten Daten zur Sauerstoffkonzentration und C02-Konzentration in der Ausatemluft des Probanden 3 werden zu einem Personal Computer (PC) 6 übertragen. Ebenso werden die Daten zur Herzfrequenz des Probanden 3 und Leistungsparameterdaten des Laufbands 2 vom Monitor 5 zum PC 6 übertragen. Im PC 6 wird der respiratorische Quotient des Probanden 3 auf der Grundlage der ermittelten Sauerstoffkonzentration und C02-Konzentration in der Ausatemluft des Probanden bestimmt. Anhand des derart ermittelten respiratorischen Quotienten des Probanden 3 werden korrigierte Laufbandleistungsdaten zum Monitor 5 des Laufbands 2 übertragen. Der Monitor 5 weist eine Steuereinheit auf, die die Leistungsparameter des Laufbands 2 anpassen kann.

In Abhängigkeit des ermittelten respiratorischen Quotienten des Probanden 3 erfolgt auf diese Art und Weise eine Anpassung der Leistungsparameter des Laufbands in einem vorgegebenen Leistungsäquivalentbereich. Damit dienen metabolische Daten des Probanden 3 zur Steuerung des Laufbands 2.

Die im Rahmen dieses Verfahrens zur Steuerung des Laufbands 2 vorgesehenen Datenübertragungen sind durch entsprechende Striche und Pfeile verdeutlicht. Die Datenübertragung kann dabei drahtlos oder drahtgebunden erfolgen.

Zusätzlich ist im Trainingsraum 1 ein Sensor 7 zur Bestimmung der Sauerstoffkonzentration und der C02-Konzentration in der Raumatmosphäre des Trainingsraums 1 vorgesehen. Dieser Sensor 7 weist zudem eine Anzeigeeinheit auf, mit der er die ermittelten Werte der Sauerstoffkonzentration und der C02-Konzentration unmittelbar anzeigen kann. Der Sensor 7 kann - anders als in der Figur 1 dargestellt - auch mit dem PC 6 verbunden sein. Auf diese Weise ist es besonders einfach möglich, aus der Sauerstoffkonzentration in der

Ausatemluft des Probanden 3 in Zusammenschau mit der ermittelten Sauerstoffkonzentration in der Raumatmosphäre des Trainingsraums 1 die von dem Probanden 3 aufgenommene Sauerstoffmenge zu bestimmen, die letztlich für die Bestimmung des respiratorischen Quotienten maßgeblich ist.

Die Überführung der Ausatemluft vom Probanden 3 zum Messmodul 4 erfolgt vorzugsweise mit einem Mundstück, das in der Figur 1 nicht gesondert dargestellt ist. In dieses Mundstück oder in einen Schlauch, der dieses Mundstück mit dem Messmodul 4 verbindet, kann ein Durchflussmesser integriert sein, um die Durchflussrate und damit die pro Zeitintervall ausgeatmete Atemluft des Probanden 3 zu bestimmen. Auf diese Weise lässt sich aus der ermittelten Sauerstoffkonzentration und CO₂-Konzentration in der Ausatemluft des Probanden 3 die jeweilige Sauerstoffmenge pro Zeil und CO₂-Menge pro Zeit in der Ausatemluft des Probanden bestimmen.

In der Figur 1 ist der PC 6 in einem Kontrollraum 8 angeordnet. Alternativ könnte der PC 6 auch direkt im Trainingsraum 1 angeordnet sein.

Die Figur 2 zeigt eine schematische Darstellung einer Variante eines Verfahrens zur Steuerung eines Trainingsgeräts. Zunächst werden Daten 10, die die Sauerstoffkonzentration und CO₂-Konzentration in der Ausatemluft eines Probanden betreffen, dazu verwendet, um eine Berechnung 11 des respiratorischen Quotienten (RQ-Wert) des Probanden zu ermitteln. Diese Berechnung 11 des RQ-Werts erfolgt typischerweise in einem PC. Anschließend erfolgt ein Abgleich 12 mit einem zuvor festgelegten Sollbereich des RQ-Werts. Dieser Sollbereich des RQ-Werts liegt im vorliegenden Fall bei 0,77 bis 0,85. Anschließend erfolgt eine Korrektur 13 unter Berücksichtigung eines Sollbereichs für die Herzfrequenz des Probanden. Auf diese Weise wird ein korrigierter Sollbereich 14 der Herzfrequenz des Probanden erreicht. Die vorgenannten Berechnungs- und Korrekturschritte erfolgen in dem PC 6, der bereits in der Figur 1 dargestellt war. Alternativ können Sie auch separat erfolgen und entsprechende Daten dem PC 6 zugeführt werden.

Vom Probanden wird - wie bereits im Zusammenhang mit der Figur 1 erläutert - die Herzfrequenz gemessen und an den PC 6 übertragen. Entsprechende Daten 15 werden nun zu einem Abgleich 16 mit dem Sollbereich der Herzfrequenz bzw. mit dem korrigierten Sollbereich der Herzfrequenz verglichen. Ausgehend von diesen Vergleichsschritten erfolgt nun eine Korrektur 17 der momentanen Leistung des Laufbands oder des anderen Trainingsgerätes, das der Proband momentan benutzt. Hierzu werden entsprechende Steuerungssignale 18 an das Laufband oder das Trainingsgerät übersandt.

Das in der Figur 2 dargestellte Verfahren berücksichtigt also sowohl den respiratorischen Quotienten des Probanden als auch dessen Herzfrequenz, um ausgehend von diesen Daten unter Berücksichtigung vorgegebener Wertebereiche eine Anpassung der Leistungsparameter des eingesetzten Trainingsgerätes zu vermitteln.

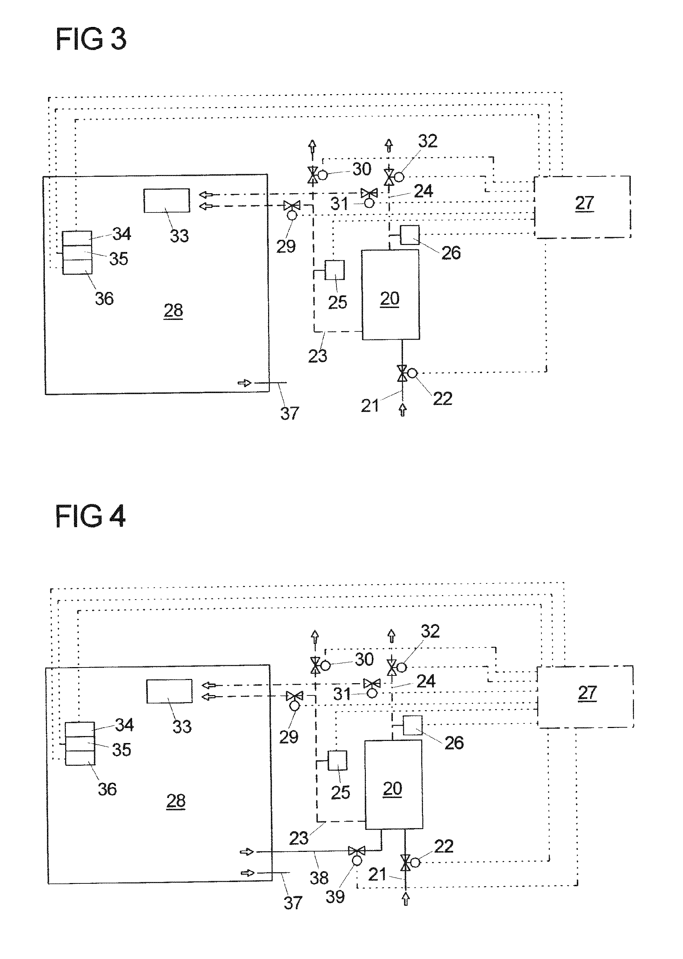

Die Figur 3 zeigt ein erstes Ausführungsbeispiel eines Raumluftsystems. Ein solches Raumluftsystem kann auch als Vorrichtung zur Bereitstellung einer definierten Raumatmosphäre in einem Trainingsraum bezeichnet werden. Kernstück des Raumluftsystems ist eine Luftzerlegungsanlage 20, die durch Druckluft 21 gespeist wird. Die Zufuhr der Druckluft 21 in die Luftzerlegungsanlage 20 kann durch ein erstes Ventil 22 unterbrochen bzw. geregelt werden. In der Luftzerlegungsanlage 20 wird die zugeführte Druckluft 21 in einen sauerstoffangereicherten Volumenstrom 23 und in einen stickstoffangereicherten Volumenstrom 24 aufgespalten. Die Sauerstoffkonzentration im sauerstoffangereicherten Volumenstrom 23 wird mittels eines ersten Sauerstoffsensors 25 überwacht. Die Sauerstoffkonzentration im stickstoffangereicherten Volumenstrom 24 wird mittels eines zweiten Sauerstoffsensors 26 überwacht. Sowohl der erste Sauerstoffsensor 25 als auch der zweite Sauerstoffsensor 26 sind mit einer Steuerungs-/Regelungseinheit 27 verbunden. Dies gilt auch für das erste Ventil 22 und sämtliche weiteren Ventilen, die nachfolgend erläutert werden.

Der sauerstoffangereicherte Volumenstrom 23 wird über eine entsprechende Rohrleitung in einen Trainingsraum 28 geleitet, der mit einer definierten Raumatmosphäre versorgt werden soll. Dabei ist ein zweites Ventil 29 vorgesehen, das der Einstellung der Durchflussrate im sauerstoffangereicherten Volumenstrom 23 dient. Ferner ist der sauerstoffangereicherte Volumenstrom 23 durch ein drittes Ventil 30 mit der umgebenden Atmosphäre verbunden, so dass über dieses dritte Ventil 30 jederzeit ein Druckausgleich in die Umgebungsluft erfolgen kann.

Gleichermaßen ist der stickstoffangereicherte Volumenstrom 24 über ein viertes Ventil 31 mit dem Trainingsraum 28 in Strömungsverbindung zu bringen. Durch Regelung des vierten Ventils 31 kann die Durchflussrate im stickstoffangereicherten Volumenstrom 24 eingestellt werden. Ferner ist ein fünftes Ventil 32 vorgesehen, mit dem nicht benötigte Anteile des stickstoffangereicherten Volumenstroms 24 in die Umgebungsatmosphäre abgelassen werden können.

Im Trainingsraum 28 ist ein Ventilator 33 als Umluftanlage vorgesehen, der der Umwälzung der unterschiedlichen Gase in der Raumatmosphäre des Trainingsraums 28 dient. Ferner sind im Trainingsraum 28 ein CO₂-Sensor 34, ein Sauerstoffsensor 35 und ein Monitor 36 angeordnet, mittels derer die aktuelle CO₂-Konzentration und die aktuelle Sauerstoffkonzentration in der Raumatmosphäre bestimmt und angezeigt werden kann. Der CO₂-Sensor 34, der Sauerstoffsensor 35 und der Monitor 36 sind mit der Steuerungs-/Regelungseinheit 27 elektronisch verbunden. Damit ist es der Steuerungs-/Regelungseinheit 27 möglich, sowohl den Sauerstoffgehalt als auch den CO₂-Gehalt im Trainingsraum 28 zu überwachen und die einzelnen Ventile des Raumluftsystems entsprechend zu regeln, um eine definierte Zufuhr des sauerstoffangereicherten Volumenstroms 23 und des stickstoffangereicherten Volumenstroms 24 in den Trainingsraum 28 zu ermöglichen. Im Trainingsraum 28 werden die beiden Volumenströme dann durch die Umluftanlage 33 vermischt, so dass sich eine einheitliche Raumatmosphäre einstellt.

Der sauerstoffangereicherte Volumenstrom 23 und der stickstoffangereicherte Volumenstrom 24 stellen die einzige Gaszufuhr in den Trainingsraum 28 dar. Damit Gas aus dem Trainingsraum 28 wieder entweichen kann, ist zudem ein Gasablass 37 vorgesehen, der den Trainingsraum 28 in die Umgebungsatmosphäre entlüftet.

Die Figur 4 zeigt ein zweites Ausführungsbeispiel eines Raumluftsystems, das sich nur in Details von dem in der Figur 3 dargestellten ersten Ausführungsbeispiel unterscheidet. Gleiche Elemente werden mit gleichen Bezugszeichen wie in der Figur 3 versehen; es wird auf die diesbezüglichen Erläuterungen zur Figur 3 verwiesen. Nachfolgend sollen lediglich die Unterschiede zum ersten Ausführungsbeispiel dargelegt werden.

Das in der Figur 4 dargestellte Ausführungsbeispiel weist neben dem Gasablass 37 eine Umluftleitung 38 auf, die über ein sechstes Ventil 39 mit der Luftzerlegungsanlage 20 verbunden ist. Auf diese Weise ist es möglich, zumindest einen Teil der in den Trainingsraum 28 befindlichen Luft wieder zur Luftzerlegungsanlage zurückzuführen und so einen Umluftbetrieb aufrechtzuerhalten. Ein derartiger Umluftbetrieb erlaubt ein noch energieärmeres Betreiben des Raumluftsystems. Denn auf diese Art und Weise entfällt das Erwärmen extern zugeführter Druckluft in der Luftzerlegungsanlage oder danach, um eine bestimmte Temperatur in dem Trainingsraum 28 zu erreichen.

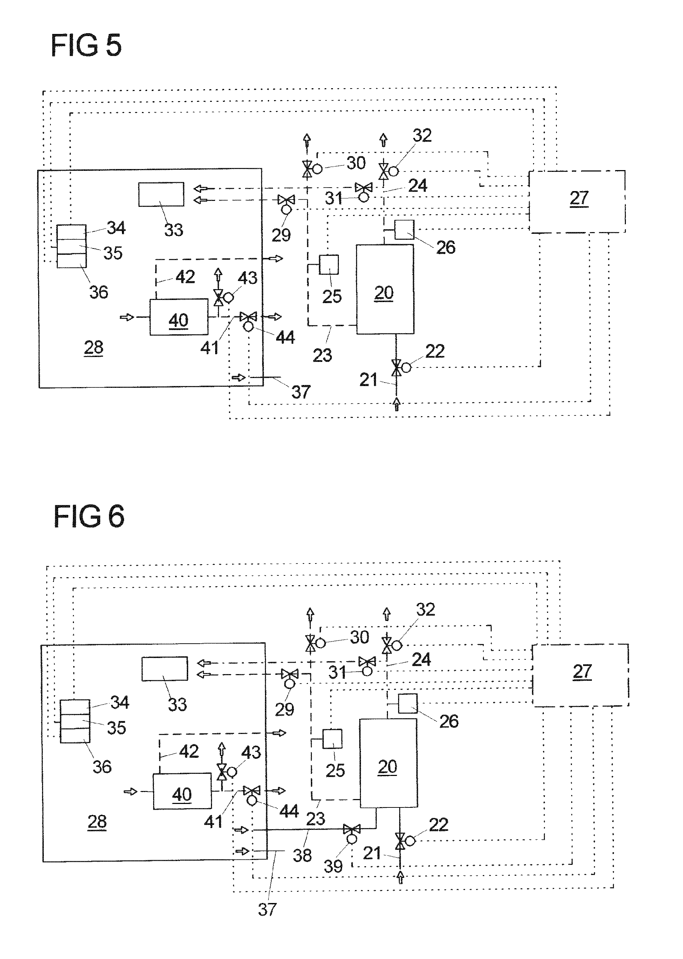

Die Figur 5 zeigt ein drittes Ausführungsbeispiel eines Umluftsystems, das wiederum dem in der Figur 3 gezeigten ersten Ausführungsbeispiel ähnelt. Gleiche Elemente werden abermals mit gleichen Bezugszeichen versehen; diesbezüglich wird auf die obigen Erläuterungen verwiesen.

Im Unterschied zu dem in der Figur 3 dargestellten Ausführungsbeispiel weist das Ausführungsbeispiel der Figur 5 eine zusätzliche Luftzerlegungsanlage 40 auf, die im Inneren des Trainingsraums 28 angeordnet ist. Diese zusätzliche Luftzerlegungsanlage 40 dient als CO₂-Abscheider. So treten Sauerstoff und Kohlendioxid in klassischerweise eingesetzten Luftzerlegungsanlagen regelmäßig gleich schnell durch entsprechende Membranen dieser Luftzerlegungsanlagen. Das heißt, es ist mit derartigen klassischen Luftzerlegungsanlagen nicht möglich, Sauerstoff und Kohlendioxid sauber voneinander zu trennen. Stickstoff braucht indes länger, um durch die in den Luftzerlegungsanlagen befindlichen Membranen hindurchzutreten. Folglich kann die zusätzliche Luftzerlegungsanlage als CO₂-Abscheider dienen, indem nur ein stickstoffangereicherter Volumenstrom 41 wieder in den Trainingsraum 28 zurückgeführt wird, während ein sauerstoffangereicherter Volumenstrom 42 aus dem Trainingsraum 28 in die umgebende Atmosphäre abgelassen wird. Denn in diesem sauerstoffangereichertem Volumenstrom findet sich auch eine höhere Konzentration von Kohlendioxid.

Um nur einen bestimmten Anteil des stickstoffangereicherten Volumenstroms 41 der zusätzlichen Luftzerlegungsanlage 40 wieder in den Trainingsraum 28 zurückzuführen, sind ein siebtes Ventil 43 und ein achtes Ventil 44 vorgesehen. Das siebte Ventil 43 dient zum Öffnen und Absperren einer Verbindung zwischen der zusätzlichen Luftzerlegungsanlage 40 und dem Inneren des Trainingsraums 28. Das achte Ventil 44 dient zum Öffnen und Absperren einer Verbindung zwischen der zusätzlichen Luftzerlegungsanlage 40 und der den Trainingsraum 28 umgebenden Außenatmosphäre.

Auch das siebte Ventil 43 und das achte Ventil 44 sind mit der Steuerungs-/Regelungseinrichtung 27 verbunden und können von dieser gesteuert bzw. geregelt werden.

Die Figur 6 zeigt ein viertes Ausführungsbeispiel eines Raumluftsystems, das einer Kombination des in der Figur 5 gezeigten dritten Ausführungsbeispiels mit dem in der Figur 4 gezeigten zweiten Ausführungsbeispiel darstellt. Das heißt, bei dem Raumluftsystem der Figur 6 ist sowohl eine zusätzliche Luftzerlegungsanlage 40 als CO₂-Abscheider vorgesehen als auch eine Umluftleitung 38, um einen Umluftbetrieb zu ermöglichen. Es wird insoweit auf die entsprechenden Erläuterungen zu den vorherigen Figuren verwiesen.

Zusammenfassend lässt sich zu den Figuren 3 bis 6 folgendes ausführen:
Das beschriebene bevorzugte Verfahren zur Erzeugung einer von der Normoxie abweichenden kontrollierten, stabilen und homogenen Atmosphäre in Räumen basiert auf der dem Raum anteilig konstant, aber auch variabel hinsichtlich Menge und Konzentration kontrollierten Einleitung eines Gasgemisches, welches zuvor in einer Luftzerlegungsanlage sowohl unter Verwendung der Technologie basierend auf der Gleichgewichtsadsorption als auch der Molekularsiebwirkung in Stickstoff bzw. stickstoffangereichertes und Sauerstoff bzw. sauerstoffangereichertes Gasgemisch separiert wurde und je nach gewählter Konzentration und Abweichung zwischen Ist- und Sollwert dem Raum zuzuführen. Die Zusammenführung der zuvor separierten Luft kann sowohl innerhalb als auch außerhalb des Raumes erfolgen, wobei ein Teil, aber auch der gesamte der in Ihre Bestandteile zerlegten Luft aus dem Raum entnommen werden kann (sechstes Ventil 39). Zur Verteilung und Schaffung einer homogenen Atmosphäre im Raum wird die Luft im Umluftbetrieb betrieben. Die Überwachung der Atmosphäre im Raum erfolgt insbesondere über Sauerstoffsensoren, welche die Messwerte an eine übergeordnete Steuerung/Regelung weiterleiten, welche je nach Abweichung zum Sollwert die Ventile 22, 29, 30, 31, 32, 39 stetig ansteuert. Alternativ können zur Überwachung des Kohlendioxidgehalts im Raum Kohlendioxidsensoren eingesetzt werden, wobei die Konzentration im Raum als auch außerhalb des Raumes über einen Monitor oder LCD- Anzeige erfolgen kann.

Der Luftdruck im Raum kann bedingt durch den Anlagenaufbau vernachlässigbar geringfügig über, als auch unterhalb des den Raum umschließenden Luftdrucks liegen. Die Verteilung der Atmosphäre im Raum kann auch über Kanäle bzw. Rohre mit oder ohne den Anschluss an Ventilatoren oder Pumpen erfolgen. Der im Raum herrschende CO₂-Gehalt, welcher sich durch im Raum befindliche Personen bedingt durch die Atmung zusätzlich erhöht, reduziert sich zum einen durch die Ausspülung bedingt durch die Zufuhr der in der Luftzerlegungsanlage erzeugten Gasgemische in den Raum, zum anderen wird bei der Rückführung der im Raum herrschenden Gasatmosphäre in die Luftzerlegungsanlage mit dem Sauerstoff, bzw. sauerstoffhaltigem Gasgemisch auch CO₂ abgeschieden. Alternativ kann auch Atemkalk zur Bindung des im Raum befindlichen CO₂ eingesetzt werden bzw. die Atmosphäre im Raum über eine zweite im Raum befindliche Luftzerlegungsanlage separiert werden wobei der CO₂ angereicherte Volumenstrom aus dem Raum abgeleitet wird. Es ist bevorzugt, dass Hypoxie oder Hyperoxie bereitgestellt werden kann, wobei der stickstoffangereicherte Volumenstrom sowohl aus dem Raum (achtes Ventil 44), als auch wieder zurück in den Raum (siebtes Ventil 43) geleitet wird. In der Nutzung des Raumes gibt es keinerlei Einschränkungen.

## Patentansprüche

1. Verfahren zur Steuerung eines Trainingsgeräts, ausgenommen Verfahren zur therapeutischen Behandlung des menschlichen oder tierischen Körpers, umfassend die folgenden Schritte:
- Bereitstellung einer definierten Raumatmosphäre in einem Trainingsraum (28), in welchem sich ein Trainingsgerät (2) befindet,
- Bestimmung eines respiratorischen Quotienten eines das Trainingsgerät (2) in dem Trainingsraum (28) benutzenden Probanden (3),
- Verwendung des respiratorischen Quotienten des Probanden (3) als Eingangsgröße zur Steuerung des Trainingsgeräts (2),
**dadurch gekennzeichnet**,
dass die Raumatmosphäre in dem Trainingsraum (28) einen konstanten Sauerstoffanteil aufweist, der im Bereich von 22 bis 32 Volumenprozent liegt, und dass ein vorgebbares Leistungsäquivalent des Trainingsgeräts (2) in einem Bereich von 50 bis 85 Watt in Abhängigkeit des respiratorischen Quotienten des Probanden (3) derart variiert wird, dass sich der respiratorische Quotient des Probanden (3) auf einen Wert in einem Bereich von 0,77 bis 0,85 einstellt.

2. Verfahren gemäß Anspruch 1, **dadurch gekennzeichnet, dass** das Leistungsäquivalent des Trainingsgeräts (2) erhöht wird, wenn der respiratorische Quotient des Probanden (3) größer als 0,85 ist.

3. Verfahren gemäß Anspruch 1 oder 2, **dadurch gekennzeichnet, dass** das Leistungsäquivalent des Trainingsgeräts (2) erniedrigt wird, wenn der respiratorische Quotient des Probanden (3) kleiner als 0,77 ist.

4. Verfahren gemäß einem der vorherigen Ansprüche, **dadurch gekennzeichnet, dass** das Leistungsäquivalent des Trainingsgeräts (2) zumindest während eines Zeitraums von 5 Minuten konstant gehalten wird.

5. Verfahren gemäß einem der vorherigen Ansprüche, **dadurch gekennzeichnet, dass** zur Bestimmung des respiratorischen Quotienten eine Messung der CO₂-Konzentration in der Ausatemluft des Probanden (3) mittels eines maskenlosen Messsystems erfolgt.

6. Verfahren gemäß Anspruch 5, **dadurch gekennzeichnet, dass** die Messung der CO₂-Konzentration in Intervallen erfolgt.

7. Verfahren gemäß Anspruch 5 oder 6, **dadurch gekennzeichnet, dass** der Proband (3) zur Messung der CO₂-Konzentration die Ausatemluft zumindest einer Ausatmung in ein Mundstück ausatmet, das mit einem CO₂-Sensor (4) verbunden ist.

8. Verfahren gemäß einem der vorherigen Ansprüche, **dadurch gekennzeichnet, dass** eine Herzfrequenz des Probanden (3) bestimmt und als zusätzliche Größe zur Steuerung des Trainingsgeräts (2) herangezogen wird.

## Claims

1. Method for controlling a training device, excluding methods for the therapeutic treatment of the human or animal body, comprising the following steps:
- providing a defined room atmosphere in a training room (28), in which a training device (2) is located,
- determining a respiratory quotient of a test subject (3) using the training device (2) in the training room (28),
- using the respiratory quotient of the test subject (3) as an input variable for controlling the training device (2),
**characterized in that**
the room atmosphere in the training room (28) has a constant oxygen content, which is in the range of from 22 to 32 percent by volume, and **in that** a predefinable equivalent power of the training device (2) is varied in a range of from 50 to 85 watt in dependence on the respiratory quotient of the test subject (3) in such a way that the respiratory quotient of the test subject (3) assumes a value in a range of from 0.77 to 0.85.

2. Method according to Claim 1, **characterized in that** the equivalent power of the training device (2) is increased if the respiratory quotient of the test subject (3) is greater than 0.85.

3. Method according to Claim 1 or 2, **characterized in that** the equivalent power of the training device (2) is reduced if the respiratory quotient of the test subject (3) is less than 0.77.

4. Method according to any of the preceding claims, **characterized in that** the equivalent power of the training device (2) is kept constant, at least during a time period of 5 minutes.

5. Method according to any of the preceding claims, **characterized in that** in order to determine the respiratory quotient, a measurement is made of the CO₂ concentration in the exhaled air of the test subject (3) by means of a mask-less measuring system.

6. Method according to Claim 5, **characterized in that** the measurement of the CO₂ concentration is carried out at intervals.

7. Method according to Claim 5 or 6, **characterized in that**, in order to measure the CO₂ concentration, the test subject (3) exhales the exhaled air of at least one exhalation into a mouthpiece which is connected to a CO₂ sensor (4).

8. Method according to any of the preceding claims, **characterized in that** a heart rate of the test subject (3) is determined and is used as an additional variable for controlling the training device (2).

## Revendications

1. Procédé de commande d'un appareil d'entraînement, sauf procédés pour le traitement thérapeutique du corps humain ou animal, comprenant les étapes suivantes:
- préparation d'une atmosphère ambiante définie dans un local d'entraînement (28), dans lequel il se trouve un appareil d'entraînement (2),
- détermination d'un quotient respiratoire d'un probant (3) utilisant l'appareil d'entraînement (2) dans le local d'entraînement (28),
- utilisation du quotient respiratoire du probant (3) comme grandeur d'entrée pour la commande de l'appareil d'entraînement (2),
caractérisé
en ce que l'atmosphère ambiante dans le local d'entraînement (28) présente une proportion d'oxygène constante, qui se situe dans la plage de 22 à 32 pour cent en volume, et en ce que l'on fait varier un équivalent de puissance pré-déterminable de l'appareil d'entraînement (2) dans une plage de 50 à 85 watts en fonction du quotient respiratoire du probant (3), de telle manière que le quotient respiratoire du probant (3) s'établisse à une valeur comprise dans une plage de 0,77 à 0,85.

2. Procédé selon la revendication 1, **caractérisé en ce que** l'on augmente l'équivalent de puissance de l'appareil d'entraînement (2), lorsque le quotient respiratoire du probant (3) est supérieur à 0,85.

3. Procédé selon la revendication 1 ou 2, **caractérisé en ce que** l'on diminue l'équivalent de puissance de l'appareil d'entraînement (2) lorsque le quotient respiratoire du probant (3) est inférieur à 0,77.

4. Procédé selon l'une quelconque des revendications précédentes, **caractérisé en ce que** l'on maintient constant l'équivalent de puissance de l'appareil d'entraînement (2) au moins pendant un intervalle de temps de 5 minutes.

5. Procédé selon l'une quelconque des revendications précédentes, **caractérisé en ce que**, pour la détermination du quotient respiratoire, on effectue une mesure de la concentration en CO₂ dans l'air expiré par le probant (3) au moyen d'un système de mesure sans masque.

6. Procédé selon la revendication 5, **caractérisé en ce que** l'on effectue la mesure de la concentration en CO₂ par intervalles.

7. Procédé selon la revendication 5 ou 6, **caractérisé en ce que** le probant (3) expire l'air expiré d'au moins une expiration dans un embout buccal, qui est relié à un détecteur de CO₂ (4), pour la mesure de la concentration en CO₂.

8. Procédé selon l'une quelconque des revendications précédentes, **caractérisé en ce que** l'on détermine une fréquence cardiaque du probant (3) et on l'utilise comme grandeur d'entrée additionnelle pour la commande de l'appareil d'entraînement (2).
